# FASCICULE DE BREVET EUROPEEN

(11) **EP 4 210 582 B1**
(45) Date de publication et mention de la délivrance du brevet: **02.07.2025**
(21) Numéro de dépôt: 21773608.1
(22) Date de dépôt: 09.09.2021
(51) Int. Cl.: A61B 6/10, G21F 3/00, G01T 1/02

(54) **PARAVENT DE RADIOPROTECTION, ADAPTÉ À ASSURER LA RADIOPROTECTION D'UN OPÉRATEUR CONTRE LES RAYONNEMENTS IONISANTS**
STRAHLENSCHUTZSCHILD ZUM SCHUTZ GEGEN IONISIERENDE STRAHLUNG
RADIATION PROTECTION PANEL AGAINST IONIZING RADIATION

(30) Priorité: 10.09.2020 FR 2009150
(43) Date de publication de la demande: 19.07.2023
(73) Titulaire: Lemer Pax, 44240 La Chapelle-sur-Erdre (FR)
(72) Inventeur: LEMER, Pierre-Marie, 44100 Nantes (FR)
(74) Mandataire: Jacobacci Coralis Harle
(86) Numéro de dépôt international: PCT/EP2021/074851
(87) Numéro de publication internationale: WO 2022/053575

(56) Documents cités:
- EP-A1- 2 422 702
- US-A- 5 981 964
- US-A1- 2013 112 897

## Description

### Domaine technique de l'invention

La présente invention concerne le domaine général des équipements de protection contre les rayonnements ionisants.

Elle concerne plus particulièrement les écrans, enceintes et paravents de radioprotection qui sont adaptés à assurer la radioprotection d'un opérateur contre les rayonnements ionisants, avantageusement dans le milieu médical ou autre.

### Etat de la technique

Dans certains domaines, les opérateurs doivent pouvoir se protéger contre une exposition aux rayonnements ionisants, par exemple les rayons X ou gamma.

C'est en particulier le cas pour le personnel soignant lors de certaines interventions dans le milieu médical, à savoir par exemple les examens du genre cathétérisme, pose de pacemaker, examens vasculaires, neurologiques ou urologiques.

L'opérateur (technicien, médecin, chirurgien ou autre) utilise alors une structure pour sa protection contre les rayonnements auxquels le patient est soumis.

Parmi les structures de protection existantes, les paravents de radioprotection sont constitués de panneaux, ou d'assemblages de panneaux, réalisés en matériaux radioprotecteurs pour assurer la radioprotection de l'opérateur.

Toutefois, en pratique, l'opérateur ne dispose souvent pas de données concrètes, objectives et fiables quant à l'efficacité radioprotectrice du paravent derrière lequel il intervient.

Or, les paravents de radioprotection actuels présentent des qualités diverses et inégales quant à leurs efficacités radioprotectrices.

Il existe par conséquent un besoin d'une solution technique qui permettrait à l'opérateur de s'informer, de manière objective et au cours de son intervention, sur l'efficacité de la protection apportée par le paravent de radioprotection qu'il utilise.

Le document US 5,981,964 A décrit un paravent radio-protecteur équipé de capteurs de rayonnement.

### Présentation de l'invention

Afin de remédier à l'inconvénient précité de l'état de la technique, la présente invention propose donc un paravent de radioprotection qui est adapté à assurer la radioprotection d'un opérateur contre les rayonnements ionisants.

Le paravent comprend une paroi radioprotectrice réalisée en matériau radioprotecteur.

Cette paroi radioprotectrice comprend deux faces opposées :
- une face intérieure, en regard de laquelle l'opérateur est destiné à se positionner, et
- une face extérieure, en regard de laquelle une source de rayonnements ionisants est destinée à être positionnée.

Selon l'invention, ledit paravent de radioprotection est équipé :
- de premiers moyens de mesure dosimétrique, implantés en regard de ladite face extérieure et adaptés à mesurer une dose de radiation extérieure reçue du côté de ladite face extérieure,
- de seconds moyens de mesure dosimétrique, implantés en regard de ladite face intérieure et adaptés à mesurer une dose de radiation intérieure reçue du côté de ladite face intérieure,
- de moyens électroniques d'affichage, adaptés à afficher des informations à destination de l'opérateur, et
- de moyens de commande, couplés auxdits moyens de mesure dosimétrique et auxdits moyens électroniques d'affichage.

Les moyens de commande comportent un module de pilotage conçu pour afficher, sur lesdits moyens électroniques d'affichage, des informations relatives au moins auxdites doses de radiation intérieure / extérieure mesurées.

En pratique, l'opérateur dispose alors d'informations / de données concrètes, objectives et fiables quant aux caractéristiques radioprotectrices du paravent derrière lequel il intervient.

Cette solution technique permet à l'opérateur de connaître, de manière objective et directe, l'efficacité de la protection conférée par le paravent de radioprotection qu'il utilise au cours de ses interventions.

D'autres caractéristiques non limitatives et avantageuses du produit conforme à l'invention, prises individuellement ou selon toutes les combinaisons techniquement possibles, sont les suivantes :
- le module de pilotage est conçu pour afficher, sur lesdits moyens électroniques d'affichage, au moins des informations choisies parmi : une valeur de la dose de radiation intérieure, une valeur de la dose de radiation extérieure, une valeur de dose de radiation différentielle (correspondant à la différence entre ladite dose de radiation extérieure et ladite dose de radiation intérieure), une valeur d'atténuation (correspondant au ratio entre ladite dose de radiation extérieure et ladite dose de radiation intérieure), lesquelles valeurs sont avantageusement en temps réel (dites encore « valeurs instantanées ») et/ou en période temporelle (dites encore « valeurs cumulées »), et lesquelles valeurs sont avantageusement exprimées en dose équivalente ;
- les premiers moyens de mesure sont portés par la face extérieure de la paroi radioprotectrice et les seconds moyens de mesure sont portés par la face intérieure de la paroi radioprotectrice ;
- les moyens électroniques d'affichage sont portés par la face intérieure de la paroi radioprotectrice et sont agencés de sorte à être accessible visuellement par l'opérateur positionné devant ladite face intérieure ;
- les moyens de commande sont couplés auxdits moyens de mesure et/ou auxdits moyens électroniques d'affichage, par le biais de moyens de connexion filaires ou sans fils ;
- le paravent de radioprotection comprend des moyens pour la fixation amovible : desdits premiers moyens de mesure dosimétrique en regard de la face extérieure de la paroi radioprotectrice, et/ou desdits seconds moyens de mesure dosimétrique en regard de la face intérieure de la paroi radioprotectrice, et/ou des moyens électroniques d'affichage en regard de la face intérieure de la paroi radioprotectrice ;
- les premiers moyens de mesure dosimétrique, et/ou les seconds moyens de mesure dosimétrique, et/ou les moyens électroniques d'affichage, sont logés chacun dans un boitier fixé de manière amovible par aimantation sur une paroi métallique du paravent ;
- les premiers moyens de mesure dosimétrique, et/ou les seconds moyens de mesure dosimétrique, et/ou les moyens électroniques d'affichage, sont logés dans un boitier fixé de manière amovible par insertion dans un logement support fixé sur le paravent ;
- les premiers moyens de mesure dosimétrique, les seconds moyens de mesure dosimétrique et les moyens électroniques d'affichage sont alimentés en électricité par des batteries portées par le paravent ;
- les premiers moyens de mesure dosimétrique, les seconds moyens de mesure dosimétrique et les moyens électroniques d'affichage sont logés chacun dans un boitier intégrant une batterie pour leur alimentation électrique, lesquels boitiers comprennent une connectique de raccordement adaptée pour la recharge desdites batteries à partir d'une source d'électricité ;
- le paravent comprend une paroi radioprotectrice comprenant un panneau frontal prolongé par au moins un panneau latéral, lesquels panneaux frontal et latéral s'étendent sur une hauteur supérieure à la hauteur de l'opérateur en position debout, les premiers moyens de mesure dosimétrique et les seconds moyens de mesure dosimétrique étant fixés sur ledit panneau latéral, sur une zone située en regard de la partie de tête dudit opérateur, et lesdits moyens électroniques d'affichage étant fixés sur ledit panneau frontal ;
- les moyens de commande comportent une sortie pour son couplage avec un ordinateur distant ;
- les moyens de commande comportent un module mémoire, conçu pour enregistrer les informations relatives aux doses de radiation intérieure / extérieure mesurées ;
- les moyens électroniques d'affichage et les moyens de commande consistent en un dispositif électronique monobloc.

La présente invention concerne encore un système dosimétrique, pour paravent de radioprotection adapté à assurer la radioprotection d'un opérateur contre les rayonnements ionisants, ledit paravent comprenant une paroi radioprotectrice réalisée en matériau radioprotecteur, ladite paroi radioprotectrice comprenant deux faces opposées (une face intérieure, en regard de laquelle l'opérateur est destiné à se positionner, et une face extérieure, en regard de laquelle une source de rayonnements ionisants est destinée à être positionnée).

Le système dosimétrique comprend :
- des premiers moyens de mesure dosimétrique, destinés à être implantés en regard de ladite face extérieure et adaptés à mesurer une dose de radiation extérieure reçue du côté de ladite face extérieure,
- des seconds moyens de mesure dosimétrique, destinés à être implantés en regard de ladite face intérieure et adaptés à mesurer une dose de radiation intérieure reçue du côté de ladite face intérieure,
- des moyens électroniques d'affichage, adaptés à afficher des informations à destination de l'opérateur, et
- des moyens de commande, couplés auxdits moyens de mesure dosimétrique et auxdits moyens électroniques d'affichage,
lesquels moyens de commande comportent un module de pilotage conçu pour afficher, sur lesdits moyens électroniques d'affichage, des informations relatives au moins auxdites doses de radiation intérieure / extérieure mesurées.

Bien entendu, les différentes caractéristiques, variantes et formes de réalisation de l'invention peuvent être associées les unes avec les autres selon diverses combinaisons dans la mesure où elles ne sont pas incompatibles ou exclusives les unes des autres.

### Description détaillée de l'invention

De plus, diverses autres caractéristiques de l'invention ressortent de la description annexée effectuée en référence aux dessins qui illustrent des formes, non limitatives, de réalisation de l'invention et où :
- la figure 1 est une vue générale, et en perspective, d'un paravent de radioprotection selon l'invention, équipé du système dosimétrique conçu notamment pour mesurer les doses de radiation intérieure / extérieure ;
- la figure 2 est une vue générale, et de côté, du paravent de radioprotection selon la figure 1, illustrant encore l'agencement du système dosimétrique ;
- la figure 3 est une vue générale, et en perspective, d'une variante de réalisation d'un paravent de radioprotection selon l'invention ;
- la figure 4 est une vue de face du paravent de radioprotection illustré sur la figure 3, orienté du côté de la face intérieure de son panneau frontal ;
- la figure 5 est une vue de face du paravent de radioprotection illustré sur la figure 3, orienté du côté de la face intérieure de son panneau latéral ;
- la figure 6 est une vue en coupe du paravent de radioprotection selon le plan de coupe 6-6 de la figure 5 ;
- la figure 7 illustre un exemple possible des informations susceptibles d'être délivrées par les moyens électroniques d'affichage du paravent de radioprotection selon l'invention.

Il est à noter que, sur ces figures, les éléments structurels et/ou fonctionnels communs aux différentes variantes peuvent présenter les mêmes références.

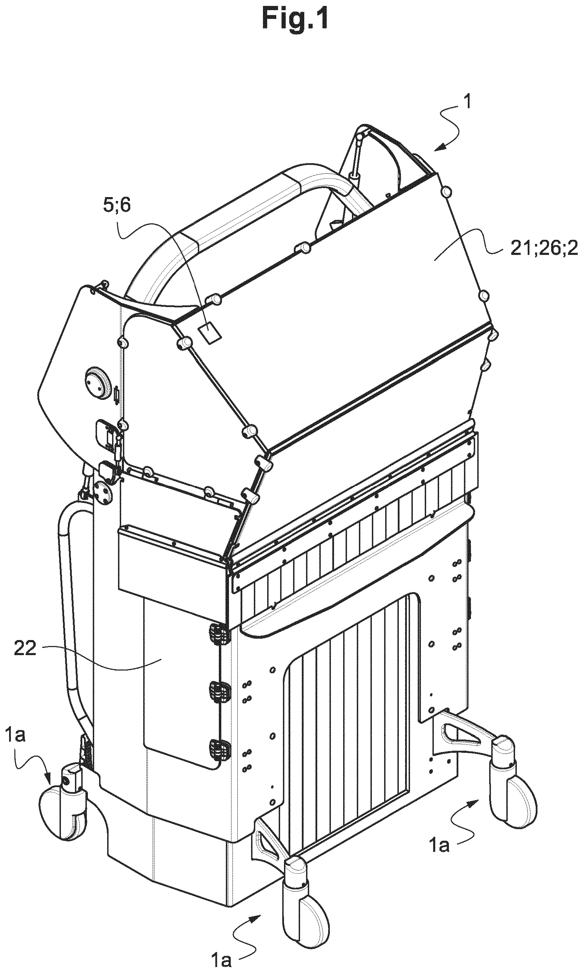

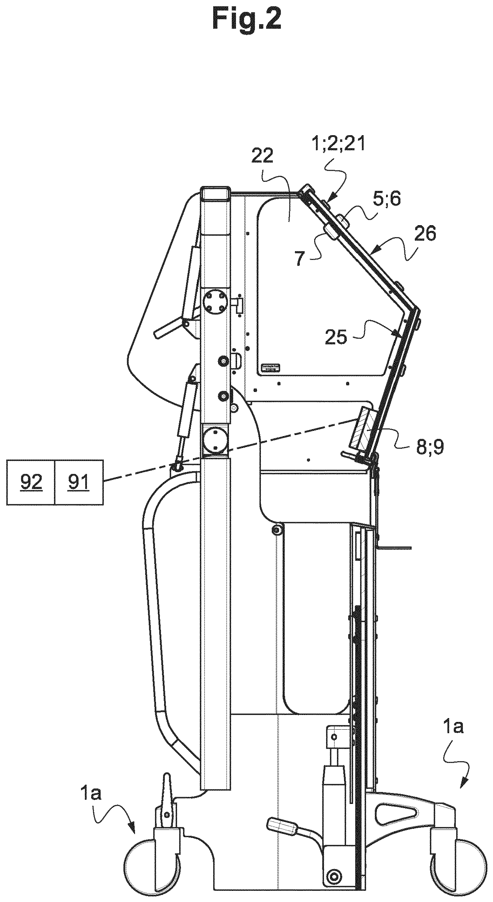

Le paravent de radioprotection 1 (dit encore « paravent 1 »), illustré sur les figures 1 et 2, est adapté à assurer la radioprotection d'un opérateur contre les rayonnements ionisants (par exemple les rayons X ou gamma).

Un tel paravent de radioprotection 1 est avantageusement utilisé dans le milieu médical, lors de certaines interventions sur des patients, à savoir par exemple lors d'examens du genre cathétérisme, pose de pacemaker, examens vasculaires, neurologiques ou urologiques.

Le paravent 1 se présente ici sous la forme d'une cabine mobile comprenant des roulettes 1a d'appui au sol pour faciliter ses déplacements.

L'opérateur (technicien, médecin, chirurgien ou autre) est destiné à se positionner derrière ce paravent de radioprotection 1, de sorte à se protéger contre les rayonnements auxquels le patient est soumis.

Pour cela, ce paravent 1 comprend une paroi radioprotectrice 2 réalisée en matériau radioprotecteur.

Ce matériau radioprotecteur, classique en soi, est par exemple choisi parmi le plomb, l'acier, une matière plastique chargée de particules métalliques radioprotectrices ou encore le verre chargé de particules métalliques radioprotectrices.

La paroi radioprotectrice 2 se compose d'un panneau ou d'un assemblage de panneaux, s'étendant généralement verticalement ou au moins approximativement verticalement sur une hauteur supérieure à la hauteur de l'opérateur en position debout.

Cette paroi radioprotectrice 2 comprend avantageusement un panneau frontal 21 qui est éventuellement prolongé par au moins un panneau latéral 22 (en l'occurrence ici par deux panneaux latéraux 22 prolongeant chacun l'un des côtés du panneau frontal 21).

La paroi radioprotectrice 2 comprend deux faces opposées :
- une face intérieure 25 (visible sur la figure 2), en regard de laquelle l'opérateur est destiné à se positionner, et
- une face extérieure 26, en regard de laquelle la source des rayonnements ionisants est destinée à se situer.

Le paravent 1 est ainsi destiné à réduire, voire à supprimer, le passage des rayonnements ionisants au travers de la paroi radioprotectrice 2. En d'autres termes, ce paravent 1 permet une réduction, voire une suppression, de la dose de radiation depuis la face extérieure 26 vers la face intérieure 25 de la paroi radioprotectrice 2.

Selon la présente invention, ce paravent 1 est équipé de moyens fonctionnels qui vont apporter à l'opérateur des données / informations concrètes, objectives et fiables quant à son efficacité radioprotectrice.

Ces moyens fonctionnels se présentent avantageusement sous la forme d'un système dosimétrique 5 qui comprend une combinaison de composants électroniques.

Le paravent de radioprotection 1 est équipé, via le système dosimétrique 5, d'un couple de moyens de mesure dosimétrique :
- des premiers moyens de mesure dosimétrique 6, adaptés à mesurer une dose de radiation extérieure Re reçue (dit encore « mesurée ») du côté de ladite face extérieure 26, et
- des seconds moyens de mesure dosimétrique 7, adaptés à mesurer une dose de radiation intérieure Ri reçue (dit encore « mesurée ») du côté de ladite face intérieure 25.

En d'autres termes, les premiers moyens de mesure dosimétrique 6 sont destinés à être interposés entre la source de rayonnements ionisants et le paravent 1, de manière à mesurer la dose de radiation extérieure Re reçue par la face extérieure 26 ; les seconds moyens de mesure dosimétrique 7 sont destinés à être positionnés en regard de la face intérieure 25, de manière à mesurer la dose de radiation Ri qui serait susceptible de traverser le paravent 1.

« Par dose de radiation », on entend avantageusement la quantité d'énergie communiquée à un milieu par un rayonnement ionisant. Cette dose de radiation est avantageusement exprimée en µSv ou mSv ou Gy (gray).

Plus précisément, par « dose de radiation », on englobe de préférence la dose absorbée, la dose équivalente, la dose efficace, le débit de dose radioactive ou le débit d'équivalent de dose.

La dose absorbée est la quantité d'énergie absorbée en un point par unité de masse de matière (inerte ou vivante). Elle s'exprime en Grays (Gy) ou en J/kg.

La dose équivalente est le produit de la dose absorbée dans un tissu ou un organe, par un facteur de pondération tenant compte de l'effet biologique lié à la nature et à l'énergie du rayonnement. Elle s'exprime en Sievert (Sv).

La dose efficace, exprimée également en Sievert, correspond à la somme des doses équivalentes pondérées délivrées par exposition interne et externe aux différents tissus et organes du corps.

Le débit de dose radioactive détermine l'intensité d'irradiation (énergie absorbée par la matière par unité de masse et de temps). Il se mesure en Gray par seconde (Gy/s).

Le débit d'équivalent de dose correspond au débit de quantité de dose absorbée, pondérée quant aux effets biologiques par des facteurs de qualité différents selon les rayonnements. Il s'exprime en Sievert par seconde (Sv/s).

En l'espèce, les premiers moyens de mesure dosimétrique 6 sont implantés en regard de la face extérieure 26. Ces premiers moyens de mesure 6 sont de préférence encore portés par cette face extérieure 26 de la paroi radioprotectrice 2.

Les seconds moyens de mesure dosimétrique 7 sont implantés en regard de la face intérieure 25. De préférence, ces seconds moyens de mesure dosimétrique 7 sont portés par la face intérieure 25 de la paroi radioprotectrice 2.

De préférence les premiers moyens de mesure dosimétrique 6 et/ou les seconds moyens de mesure dosimétrique 7 sont intégrés chacun dans un boitier support qui est fixé de manière amovible sur le paravent 1, ou qui est porté de manière amovible par le paravent 1. Les boitiers correspondants peuvent par exemple être fixés par aimantation sur une partie métallique du châssis du paravent 1 ; en variante, ils peuvent être déposés dans des logements/réceptacles de réception adaptés, eux-mêmes fixés de manière appropriée sur le paravent 1.

Les premiers moyens de mesure dosimétrique 6 et les seconds moyens de mesure dosimétrique 7 sont avantageusement implantés à une hauteur, par rapport au sol, allant de 1500 à 1900, de préférence allant de 1600 à 1800 mm (correspondant avantageusement à une hauteur de la tête de l'opérateur, en particulier de ses yeux ou encore de son lobe temporal situé du côté de la source radioactive).

Les premiers moyens de mesure dosimétrique 6 et les seconds moyens de mesure dosimétrique 7 peuvent être disposés en vis-à-vis (c'est-à-dire l'un en face de l'autre) et prendre en sandwich la paroi radioprotectrice 2 ; de manière alternative, les premiers moyens de mesure dosimétrique 6 et les seconds moyens de mesure dosimétrique 7 peuvent être décalés.

Ces premiers moyens de mesure dosimétrique 6 et seconds moyens de mesure dosimétrique 7 peuvent être implantés sur l'un des côtés du panneau frontal 21 (comme illustré sur les figures 1 et 2), ou sur l'un des panneaux latéraux 22 (en particulier sur le panneau latéral destiné à se situer du côté de la source radioactive).

Pour mesurer de telles doses de radiation, les moyens de mesure dosimétrique 6, 7 consistent avantageusement en au moins deux dispositifs électroniques individuels qui sont chacun aptes à mesurer / déterminer une exposition externe aux rayonnements ionisants de la dose reçue pour le corps entier.

De tels moyens de mesure dosimétrique 6, 7 sont avantageusement choisis parmi les dosimètres classiques en soi, de préférence les dosimètres électroniques.

Ces dosimètres consistent de préférence en des dosimètres opérationnels ou actifs, aptes à mesurer / collecter la dose de radiation reçue en temps réel.
Ils peuvent être du type compteur de radiations Geiger-Muller.

Les boitiers supports des premiers et seconds moyens de mesure dosimétrique 6, 7 intègrent avantageusement une batterie pour assurer leur autonomie en énergie. Et les boitiers en question comprennent une connectique de raccordement adaptée pour la recharge de la batterie à partir d'une source d'électricité. Cette opération de recharge est facilitée par le montage amovible des boitiers sur le paravent 1.

Pour la mise en œuvre de ces moyens de mesure dosimétrique 6, 7, comme illustré sur la figure 2, le paravent de radioprotection 1 est encore équipé :
- de moyens électroniques d'affichage 8, adaptés à afficher des informations / données à destination de l'opérateur, et
- de moyens de commande 9, couplés aux moyens de mesure dosimétrique 6, 7 (pour le traitement des données collectées) et aux moyens électroniques d'affichage 8 (pour piloter les informations diffusées à destination de l'opérateur).

Les moyens électroniques d'affichage 8 et les moyens de commande 9 sont avantageusement logés dans un même boitier support qui intègre avantageusement une batterie, pour assurer une autonomie en énergie.

Ici encore, le boitier correspondant comprend une connectique de raccordement adaptée pour la recharge de la batterie à partir d'une source d'électricité.

Selon une variante de réalisation, le paravent 1 peut comporter une batterie rechargeable adaptée pour alimenter en électricité les moyens de mesure dosimétrique 6, 7, les moyens électroniques d'affichage 8 et les moyens de commande 9 au moyen d'un réseau filaire.

De préférence, les moyens électroniques d'affichage 8, avec éventuellement les moyens de commande 9, sont portés par la face intérieure 25 de la paroi radioprotectrice 2. Leur boitier support peut être porté de manière amovible par le paravent 1, par aimantation ou par intégration dans un logement/réceptacle support ; Ce montage amovible du boitier sur le paravent 1 facilite l'opération de recharge de la batterie intégrée.

Ils sont en outre agencés de sorte à être accessible visuellement par l'opérateur positionné devant cette face intérieure 25 au cours de son intervention.

De tels moyens électroniques d'affichage 8 se présentent avantageusement sous la forme d'un périphérique de sortie, par exemple un écran (ou moniteur).

Les moyens de commande 9 consistent avantageusement en une unité informatique / électronique, par exemple un ordinateur, qui comprend :
- une unité de traitement, dite encore « unité de calcul » ou « processeur », et
- des moyens de stockage de données contenant une base de données et au moins un programme d'ordinateur.

La connexion des moyens de commande 9 avec, d'une part, les moyens de mesure dosimétrique 6, 7 et, d'autre part, les moyens électroniques d'affichage 8, est de préférence mise en œuvre par le biais de moyens de connexion sans fils (Wifi, Bluetooth, ZigBee, etc.), non représentés. En variante, cette connexion peut être filaire.

Selon un mode de réalisation préféré, les moyens électroniques d'affichage 8 et les moyens de commande 9 consistent en un dispositif électronique monobloc (boîtier commun), par exemple du type tablette, éventuellement une tablette tactile.

Dans le cas de moyens de connexion sans fils, les moyens de mesure dosimétrique 6, 7 sont avantageusement équipés respectivement d'un module de communication émetteur / récepteur sans fils.

Les moyens de commande 9 comportent un module de pilotage 91 conçu pour afficher, sur les moyens électroniques d'affichage 8, des informations / données relatives au moins aux doses de radiation intérieure Ri / extérieure Re mesurées.

Un tel module de pilotage 91 est de préférence choisi parmi les programmes d'ordinateur comprenant des instructions qui, lorsque le programme est exécuté par un ordinateur, conduisent ledit ordinateur à afficher, sur les moyens électroniques d'affichage 8, des informations / données relatives au moins aux doses de radiation intérieure Ri / extérieure Re mesurées.

Par « informations / données relatives au moins aux doses de radiation intérieure Ri / extérieure Re mesurées », on englobe avantageusement :
- les valeurs des doses de radiation intérieure Ri / extérieure Re mesurées, en tant que telles, affichées directement après leurs mesures respectives, et/ou
- des valeurs dérivées depuis ces doses de radiation intérieure Ri / extérieure Re mesurées, issues d'un traitement par ce même module de pilotage 91.

De préférence, le module de pilotage 91 est conçu pour afficher, sur les moyens électroniques d'affichage 8, au moins des informations choisies parmi :
- la valeur Ri de la dose de radiation intérieure, collectée par les seconds moyens de mesure dosimétrique 7 (implantés en regard de ladite face intérieure 25),
- la valeur Re de la dose de radiation extérieure, collectée par les premiers moyens de mesure dosimétrique 6 (implantés en regard de la face extérieure 26),
- une valeur de dose de radiation différentielle, correspondant à la différence entre ladite dose de radiation extérieure Re et ladite dose de radiation intérieure Ri,
- une valeur de coefficient d'atténuation du paravent 1,
- une valeur d'atténuation, correspondant au ratio entre ladite dose de radiation extérieure (Re) et ladite dose de radiation intérieure (Ri), avantageusement exprimée en pourcentage de ladite dose de radiation extérieure (Re).

Les valeurs affichées sur les moyens électroniques d'affichage 8 comprennent avantageusement :
- des valeurs en temps réel ou en instantanées (mode « temps réel »), et/ou
- des valeurs sur une période temporelle ou cumulées (mode « suivi »), par exemple sur le temps d'un examen ou d'une intervention, intéressantes notamment pour le suivi dosimétrique.

La valeur cumulée peut être individualisée pour un utilisateur donnée, avec un moyen de reconnaissance de l'utilisateur (badge, etc.).

Ces valeurs affichées sont avantageusement exprimées en dose équivalente.

Les moyens de commande 9 comportent un module mémoire 92, conçu pour enregistrer les informations / données relatives aux doses de radiation intérieure Ri / extérieure Re mesurées.

Un tel module mémoire 92 est de préférence choisi parmi les programmes d'ordinateur comprenant des instructions qui, lorsque le programme est exécuté par un ordinateur, conduisent ledit ordinateur à enregistrer les informations / les données relatives aux doses de radiation intérieure Ri / extérieure Re mesurées au sein des moyens de stockage de données.

Les moyens de commande 9 peuvent également comporter une sortie (non représentée), par exemple sous la forme d'un bus informatique (par exemple un port USB), pour connecter un périphérique informatique.

Ce couplage des moyens de commande 9, filaire ou sans fil, est avantageusement réalisé avec un ordinateur distant, par exemple sous la forme d'un serveur.

Cette sortie est utile pour l'échange de données (notamment les informations relatives aux doses de radiation intérieure Ri / extérieure Re mesurées) avec l'ordinateur distant dans le cadre d'un suivi dosimétrique.

De manière générale, le paravent de radioprotection 1 est équipé du système dosimétrique 5, dès l'origine ou rapporté a posteriori.

Dans une configuration « rapporté », le système dosimétrique 5 consiste avantageusement un équipement « autonome » qui est adapté à être rapporté sur un paravent de radioprotection 1 existant pour son amélioration / sa mise au jour.

Encore de manière générale, les différents composants du système de dosimétrique 5 sont portés par le paravent de radioprotection 1, par le biais de moyens d'assemblage (éventuellement via des organes supports dédiés).

En fonctionnement, le paravent de radioprotection 1 est interposé entre l'opérateur et la source de rayonnements ionisants.

L'opérateur se positionne alors derrière le paravent 1, en regard de sa face intérieure 25.

Au cours de l'intervention, le système dosimétrique 5 collecte les doses de radiation intérieure Ri / extérieure Re.

Le système dosimétrique 5 assure également le traitement des données collectées par le biais des moyens de commande 9, et délivre les données relatives au moins aux doses de radiation intérieure Ri / extérieure Re via les moyens électroniques d'affichage 8.

Tout au long de son intervention, l'opérateur peut ainsi visualiser les données concrètes, objectives et fiables, en mode « temps réel » et/ou en mode « suivi », quant à l'efficacité radioprotectrice de son paravent de radioprotection 1.

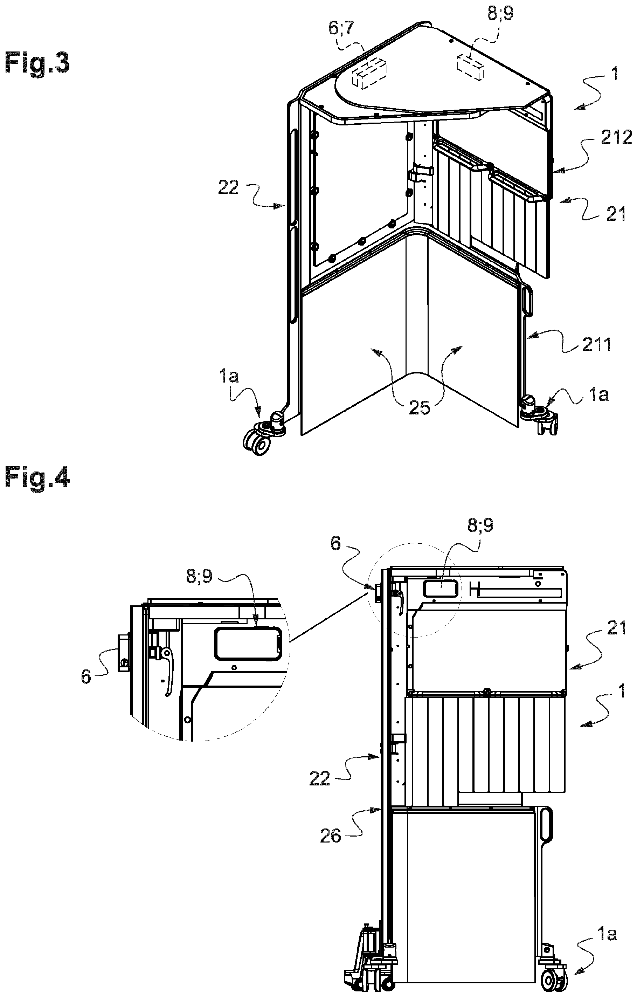

Les figures 3 à 6 illustrent une variante de réalisation d'un paravent 1 selon l'invention, en forme de cabine mobile montée sur roulettes 1a.

Ici encore, ce paravent 1 comprend une paroi radioprotectrice 2 réalisée en matériau radioprotecteur.

Ce matériau radioprotecteur, classique en soi, est par exemple choisi parmi le plomb, l'acier, une matière plastique chargée de particules métalliques radioprotectrices ou encore le verre chargé de particules métalliques radioprotectrices.

La paroi radioprotectrice 2 se compose d'un panneau ou d'un assemblage de panneaux, s'étendant généralement verticalement ou au moins approximativement verticalement sur une hauteur supérieure à la hauteur de l'opérateur en position debout.

Cette paroi radioprotectrice 2 comprend ici un panneau frontal 21 qui est prolongé sur l'un de ses côtés par un panneau latéral 22.

Le panneau frontal 21 comprend :
- une partie inférieure 211 fixe par rapport au panneau latéral 22 et s'étendant selon un angle de l'ordre de 90° par rapport à ce dernier, et
- une partie supérieure 212, mobile, montée pivotante autour d'un axe vertical sur le côté en regard du panneau latéral 22.

La paroi radioprotectrice 2, formée du panneau frontal 21 et du panneau latéral 22 comprend deux faces opposées :
- une face intérieure 25, en regard de laquelle l'opérateur est destiné à se positionner, et
- une face extérieure 26, en regard de laquelle la source des rayonnements ionisants est destinée à se situer.

Comme pour le mode de réalisation illustré sur les figures 1 et 2, le paravent 1 illustré sur les figures 3 à 6 comprend :
- des premiers moyens de mesure dosimétrique 6, adaptés à mesurer une dose de radiation extérieure Re reçue (dit encore « mesurée ») du côté de la face extérieure 26,
- des seconds moyens de mesure dosimétrique 7, adaptés à mesurer une dose de radiation intérieure Ri reçue (dit encore « mesurée ») du côté de la face intérieure 25,
- des moyens électroniques d'affichage 8, adaptés à afficher des informations / données à destination de l'opérateur, et
- des moyens de commande 9, couplés aux moyens de mesure dosimétrique 6, 7 (pour le traitement des données collectées) et aux moyens électroniques d'affichage 8 (pour piloter les informations diffusées à destination de l'opérateur).

Les premiers moyens de mesure dosimétrique 6 et les seconds moyens de mesure dosimétrique 7 sont ici disposés en vis-à-vis (c'est-à-dire l'un en face de l'autre) dans la partie supérieure du panneau latéral 22, dans une zone située à proximité de la tête de l'opérateur, et plus particulièrement en regard du lobe temporal de l'opérateur situé du côté de la source radioactive.

De préférence les premiers moyens de mesure dosimétrique 6 et les seconds moyens de mesure dosimétrique 7 sont intégrés chacun dans un boitier support qui est fixé ou porté de manière amovible sur le paravent 1. Les boitiers correspondants peuvent par exemple être fixés par aimantation sur une partie métallique du châssis du paravent ; en variante, ils peuvent être déposés dans des logements de réception adaptés, eux-mêmes fixés de manière appropriée sur le paravent 1.

Les boitiers supports des premiers et seconds moyens de mesure dosimétrique 6, 7 intègrent avantageusement une batterie pour assurer leur autonomie en énergie. Et les boitiers en question comprennent une connectique de raccordement adaptée pour la recharge de la batterie à partir d'une source d'électricité. Cette opération de recharge est facilitée par le montage amovible des boitiers sur le paravent 1 (les boitiers peuvent ainsi être désolidarisés du paravent pour être connectés à un chargeur de batteries adapté)..

Les moyens électroniques d'affichage 8 et les moyens de commande 9 sont logés dans un même boitier support fixé dans la partie supérieure du panneau frontal 21, côté face intérieure 25 du paravent 1.

Ce boitier support est avantageusement porté de manière amovible par le paravent 1, par aimantation ou par intégration dans un logement support.

Ce boitier support intègre avantageusement une batterie, pour assurer une autonomie en énergie ; et il comprend une connectique de raccordement adaptée pour la recharge de la batterie à partir d'une source d'électricité. Cette opération de recharge est facilitée par le montage amovible du boitier sur le paravent 1.

Le pilotage des moyens électroniques d'affichage 8 par les moyens de commande 9 à partir des informations reçues des moyens de mesure dosimétrique 6 et 7 est réalisé de la manière décrite ci-dessus en relation avec le mode de réalisation des figures 1 et 2.

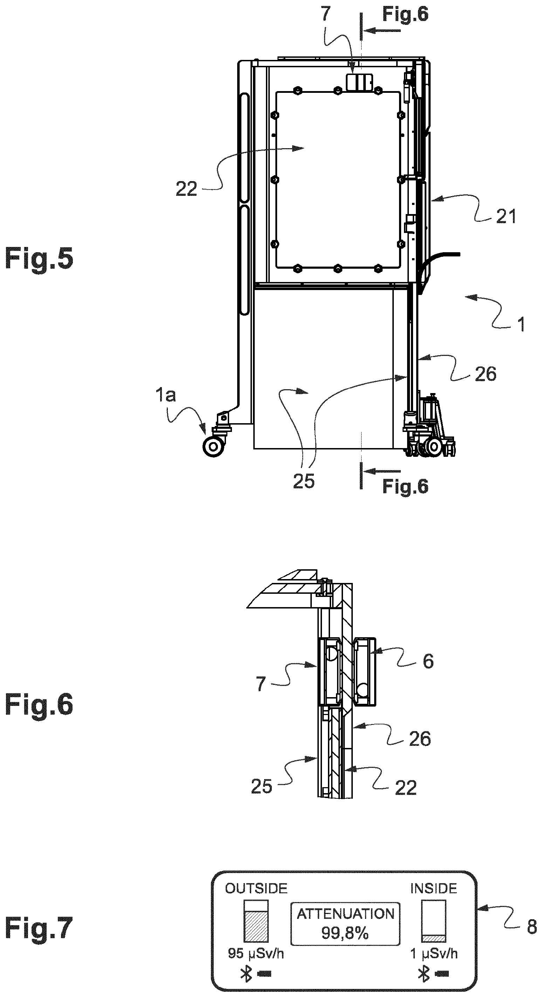

La figure 7 illustre un exemple possible d'informations susceptibles d'être délivrées par les moyens électroniques d'affichage 8.

La dose de radiation extérieure Re détectée par les premiers moyens de mesure dosimétrique 6 apparait ici sur la gauche de l'écran. La dose de radiation intérieure Ri détectée par les seconds moyens de mesure dosimétrique 7 apparait sur la droite de l'écran ; et la valeur d'atténuation de la radiation est affichée en partie centrale.

L'écran peut aussi afficher d'autres informations par exemple relatives à la charge des batteries des moyens de mesure dosimétrique 6 et 7, et au fonctionnement correct de la connexion sans fil.

## Revendications

1. Paravent de radioprotection, adapté à assurer la radioprotection d'un opérateur contre les rayonnements ionisants,
lequel paravent comprend une paroi radioprotectrice (2) réalisée en matériau radioprotecteur,
laquelle paroi radioprotectrice comprend deux faces opposées :
- une face intérieure (25), en regard de laquelle l'opérateur est destiné à se positionner, et
- une face extérieure (26), en regard de laquelle une source de rayonnements ionisants est destinée à être positionnée,
**caractérisé en ce que** ledit paravent de radioprotection (1) est équipé :
- de premiers moyens de mesure dosimétrique (6), implantés en regard de ladite face extérieure (26) et adaptés à mesurer une dose de radiation extérieure (Re) reçue du côté de ladite face extérieure (26),
- de seconds moyens de mesure dosimétrique (7), implantés en regard de ladite face intérieure (25) et adaptés à mesurer une dose de radiation intérieure (Ri) reçue du côté de ladite face intérieure (25),
- de moyens électroniques d'affichage (8), adaptés à afficher des informations à destination de l'opérateur, et
- de moyens de commande (9), couplés auxdits moyens de mesure dosimétrique (6, 7) et auxdits moyens électroniques d'affichage (8),
lesquels moyens de commande (9) comportent un module de pilotage (91) conçu pour afficher, sur lesdits moyens électroniques d'affichage (8), des informations relatives au moins auxdites doses de radiation intérieure (Ri) / extérieure (Re) mesurées.

2. Paravent de radioprotection selon la revendication 1, **caractérisé en ce que** le module de pilotage (91) est conçu pour afficher, sur lesdits moyens électroniques d'affichage (8), au moins des informations choisies parmi :
- une valeur de la dose de radiation intérieure (Ri),
- une valeur de la dose de radiation extérieure (Re),
- une valeur de dose de radiation différentielle, correspondant à la différence entre ladite dose de radiation extérieure (Re) et ladite dose de radiation intérieure (Ri), et
- une valeur d'atténuation, correspondant au ratio entre ladite dose de radiation extérieure (Re) et ladite dose de radiation intérieure (Ri).

3. Paravent de radioprotection selon l'une quelconque des revendications 1 ou 2, **caractérisé en ce que** les premiers moyens de mesure dosimétrique (6) sont portés par la face extérieure (26) de la paroi radioprotectrice (2) et les seconds moyens de mesure dosimétrique (7) sont portés par la face intérieure (25) de la paroi radioprotectrice (2).

4. Paravent de radioprotection selon l'une quelconque des revendications 1 à 3, **caractérisé en ce que** les moyens électroniques d'affichage (8) sont portés par la face intérieure (25) de la paroi radioprotectrice (2) et sont agencés de sorte à être accessible visuellement par l'opérateur positionné devant ladite face intérieure (25).

5. Paravent de radioprotection selon l'une quelconque des revendications 1 à 4, **caractérisé en ce que** les moyens de commande (9) sont couplés auxdits moyens de mesure dosimétrique (6, 7) et/ou auxdits moyens électroniques d'affichage (8), par le biais de moyens de connexion filaires ou sans fils.

6. Paravent de radioprotection selon l'une quelconque des revendications 1 à 5, **caractérisé en ce qu'**il comprend des moyens pour la fixation amovible :
- desdits premiers moyens de mesure dosimétrique (6) en regard de la face extérieure (26) de la paroi radioprotectrice (2), et/ou
- desdits seconds moyens de mesure dosimétrique (7) en regard de la face intérieure (25) de la paroi radioprotectrice (2), et/ou
- des moyens électroniques d'affichage (8) en regard de la face intérieure (25) de la paroi radioprotectrice (2).

7. Paravent de radioprotection selon la revendication 6, **caractérisé en ce que** lesdits premiers moyens de mesure dosimétrique (6), et/ou lesdits seconds moyens de mesure dosimétrique (7), et/ou lesdits moyens électroniques d'affichage (8), sont logés chacun dans un boitier fixé de manière amovible par aimantation sur une paroi métallique dudit paravent (1).

8. Paravent de radioprotection selon la revendication 6, **caractérisé en ce que** lesdits premiers moyens de mesure dosimétrique (6), et/ou lesdits seconds moyens de mesure dosimétrique (7), et/ou lesdits moyens électroniques d'affichage (8), sont logés dans un boitier fixé de manière amovible par insertion dans un logement support fixé sur ledit paravent (1).

9. Paravent de radioprotection selon l'une quelconque des revendications 1 à 8, **caractérisé en ce que** lesdits premiers moyens de mesure dosimétrique (6), lesdits seconds moyens de mesure dosimétrique (7) et lesdits moyens électroniques d'affichage (8) sont alimentés en électricité par des batteries portées par ledit paravent (1).

10. Paravent de radioprotection selon la revendication 9, **caractérisé en ce que** lesdits premiers moyens de mesure dosimétrique (6), lesdits seconds moyens de mesure dosimétrique (7) et lesdits moyens électroniques d'affichage (8) sont logés chacun dans un boitier intégrant une batterie pour leur alimentation électrique, lesquels boitiers comprennent une connectique de raccordement adaptée pour la recharge desdites batteries à partir d'une source d'électricité.

11. Paravent de radioprotection selon l'une quelconque des revendications 1 à 10, **caractérisé en ce qu'**il comprend une paroi radioprotectrice (2) comprenant un panneau frontal (21) prolongé par au moins un panneau latéral (22), lesquels panneaux frontal (21) et latéral (22) s'étendent sur une hauteur supérieure à la hauteur dudit opérateur en position debout,
lesdits premiers moyens de mesure dosimétrique (6) et lesdits seconds moyens de mesure dosimétrique (7) étant fixés sur ledit panneau latéral (22), sur une zone située en regard de la partie de tête dudit opérateur,
et lesdits moyens électroniques d'affichage (8) étant fixés sur ledit panneau frontal (21).

12. Paravent de radioprotection selon l'une quelconque des revendications 1 à 11, **caractérisé en ce que** les moyens de commande (9) comportent une sortie pour son couplage avec un ordinateur distant.

13. Paravent de radioprotection selon l'une quelconque des revendications 1 à 12, **caractérisé en ce que** les moyens de commande (9) comportent un module mémoire (92), conçu pour enregistrer les informations relatives aux doses de radiation intérieure (Ri) / extérieure (Re) mesurées.

14. Paravent de radioprotection selon l'une quelconque des revendications 1 à 13, **caractérisé en ce que** les moyens électroniques d'affichage (8) et les moyens de commande (9) consistent en un dispositif électronique monobloc.

15. Système dosimétrique, pour paravent de radioprotection (1) adapté à assurer la radioprotection d'un opérateur contre les rayonnements ionisants,
ledit paravent comprenant une paroi radioprotectrice (2) réalisée en matériau radioprotecteur, ladite paroi radioprotectrice (2) comprenant deux faces opposées : une face intérieure (25), en regard de laquelle l'opérateur est destiné à se positionner, et une face extérieure (26), en regard de laquelle une source de rayonnements ionisants est destinée à être positionnée,
lequel système dosimétrique (5) comprend :
- des premiers moyens de mesure dosimétrique (6), destinés à être implantés en regard de ladite face extérieure (26) et adaptés à mesurer une dose de radiation extérieure (Re) reçue du côté de ladite face extérieure (26),
- des seconds moyens de mesure dosimétrique (7), destinés à être implantés en regard de ladite face intérieure (25) et adaptés à mesurer une dose de radiation intérieure (Ri) reçue du côté de ladite face intérieure (25),
- des moyens électroniques d'affichage (8), adaptés à afficher des informations à destination de l'opérateur, et
- des moyens de commande (9), couplés auxdits moyens de mesure dosimétrique et auxdits moyens électroniques d'affichage (8),
lesquels moyens de commande (9) comportent un module de pilotage (91) conçu pour afficher, sur lesdits moyens électroniques d'affichage (8), des informations relatives au moins auxdites doses de radiation intérieure (Ri) / extérieure (Re) mesurées.

## Patentansprüche

1. Strahlenschutzschild, dazu ausgelegt, den Strahlenschutz eines Bedieners vor ionisierenden Strahlen sicherzustellen,
wobei der Strahlenschutzschild eine aus einem Strahlenschutzmaterial gefertigte Strahlenschutzwand (2) aufweist,
wobei die Strahlenschutzwand zwei entgegengesetzte Seiten aufweist:
- eine Innenseite (25), gegenüber der der Bediener Position beziehen soll, und
- eine Außenseite (26), gegenüber der die Quelle ionisierender Strahlen positioniert sein soll,
**dadurch gekennzeichnet, daß** der Strahlenschutzschild (1) mit
- ersten dosimetrischen Meßmitteln (6), die gegenüber der Außenseite (26) angeordnet sind und dazu ausgelegt sind, eine an der Außenseite (26) empfangene äußere Strahlungsdosis (Re) zu messen,
- zweiten dosimetrischen Meßmitteln (7), die gegenüber der Innenseite (25) angeordnet sind und dazu ausgelegt sind, eine an der Innenseite (25) empfangene äußere Strahlungsdosis (Ri) zu messen,
- elektronischen Anzeigemitteln (8), die dazu ausgelegt sind, für den Bediener bestimmte Informationen anzuzeigen, und
- mit den dosimetrischen Meßmitteln (6, 7) und den elektronischen Anzeigemitteln (8) verbundenen Steuerungsmitteln (9)
ausgestattet ist,
wobei die Steuerungsmittel (9) ein Steuerungsmodul (91) aufweisen, das dazu ausgelegt ist, auf den elektronischen Anzeigemitteln (8) mindestens auf die gemessenen inneren und äußeren Strahlendosen (Ri, Re) bezogene Informationen anzuzeigen.

2. Strahlenschutzschild gemäß Anspruch 1, **dadurch gekennzeichnet, daß** das Steuerungsmodul (91) dazu ausgelegt ist, auf den besagten elektronischen Anzeigemitteln (8) mindestens Informationen anzuzeigen, die aus den folgenden ausgewählt sind:
- einen Wert der inneren Strahlendosis (Ri),
- einen Wert der äußeren Strahlendosis (Re),
- einen Wert differentieller Strahlendosis, der der Differenz zwischen der äußeren Strahlendosis (Re) und der inneren Strahlendosis (Ri) entspricht, und
- einen Dämpfungswert, der dem Verhältnis zwischen der äußeren Strahlendosis (Re) und der inneren Strahlendosis (Ri) entspricht.

3. Strahlenschutzschild gemäß einem der Ansprüche 1 oder 2, **dadurch gekennzeichnet, daß** die ersten dosimetrischen Meßmittel (6) von der Außenseite (26) der Strahlenschutzwand (2) und die zweiten dosimetrischen Meßmittel (7) von der Innenseite (25) der Strahlenschutzwand (2) getragen werden.

4. Strahlenschutzschild gemäß einem der Ansprüche 1 bis 3, **dadurch gekennzeichnet, daß** die elektronischen Anzeigemittel (8) von der Innenseite (25) der Strahlenschutzwand (2) getragen werden und so angeordnet sind, daß sie für den vor der Innenseite (25) positionierten Bediener sichtmäßig zugänglich sind.

5. Strahlenschutzschild gemäß einem der Ansprüche 1 bis 4, **dadurch gekennzeichnet, daß** die Steuerungsmittel (9) mit den dosimetrischen Meßmitteln (6, 7) und/oder den elektronischen Anzeigemitteln (9) mittels drahtgebundener oder drahtloser Verbindungsmittel verbunden sind.

6. Strahlenschutzschild gemäß einem der Ansprüche 1 bis 5, **dadurch gekennzeichnet, daß** er Mittel für die abnehmbare Befestigung
- der ersten dosimetrischen Meßmittel (6) gegenüber der Außenseite (26) der Strahlenschutzwand (2) und/oder
- der zweiten dosimetrischen Meßmittel (7) gegenüber der Innenseite (25) der Strahlenschutzwand (2) und/oder
- der elektronischen Anzeigemittel (8) gegenüber der Innenseite (25) der Strahlenschutzwand (2)
aufweist.

7. Strahlenschutzschild gemäß Anspruch 6, **dadurch gekennzeichnet, daß** die ersten dosimetrischen Meßmittel (6) und/oder die zweiten dosimetrischen Meßmittel (7) und/oder die elektronischen Anzeigemittel (8) jeweils in einem Kasten untergebracht sind, der durch Magnetisierung auf einer Metallwand des Schilds (1) abnehmbar befestigt ist.

8. Strahlenschutzschild gemäß Anspruch 6, **dadurch gekennzeichnet, daß** die ersten dosimetrischen Meßmittel (6) und/oder die zweiten dosimetrischen Meßmittel (7) und/oder die elektronischen Anzeigemittel (8) in einem Kasten untergebracht sind, der durch Einsetzen in einen auf dem Schild (1) befestigten Aufnahmeträger abnehmbar befestigt ist.

9. Strahlenschutzschild gemäß einem der Ansprüche 1 bis 8, **dadurch gekennzeichnet, daß** die ersten dosimetrischen Meßmittel (6) und/oder die zweiten dosimetrischen Meßmittel (7) und/oder die elektronischen Anzeigemittel (8) durch vom Schild (1) getragene Batterien mit Strom versorgt werden.

10. Strahlenschutzschild gemäß Anspruch 9, **dadurch gekennzeichnet, daß** die ersten dosimetrischen Meßmittel (6) und/oder die zweiten dosimetrischen Meßmittel (7) und/oder die elektronischen Anzeigemittel (8) jeweils in einem Kasten untergebracht sind, der eine Batterie für deren Stromversorgung enthält, wobei die Kästen eine Anschlußverbindung aufweisen, die für das Aufladen der Batterien von einer Stromquelle aus ausgelegt ist.

11. Strahlenschutzschild gemäß einem der Ansprüche 1 bis 10, **dadurch gekennzeichnet, daß** er eine Strahlenschutzwand (2) aufweist, die eine durch mindestens eine Seitenplatte (22) verlängerte Frontplatte (21) aufweist, wobei sich die Frontplatte (21) und die Seitenplatten (22) über eine Höhe erstrecken, die größer als die Größe des aufrecht stehenden Bedieners ist,
wobei die ersten dosimetrischen Meßmittel (6) und die zweiten dosimetrischen Meßmittel (7) auf der Seitenplatte (22) in einer Zone befestigt sind, die gegenüber dem Kopfteil des Bedieners liegt,
und wobei die elektronischen Anzeigemittel (8) auf der Frontplatte (21) befestigt sind.

12. Strahlenschutzschild gemäß einem der Ansprüche 1 bis 11, **dadurch gekennzeichnet, daß** die Steuerungsmittel (9) einen Ausgang für deren Verbindung mit einem abgelegenen Computer aufweisen.

13. Strahlenschutzschild gemäß einem der Ansprüche 1 bis 12, **dadurch gekennzeichnet, daß** die Steuerungsmittel (9) ein Speichermodul (92) aufweisen, das dazu ausgelegt ist, die Informationen bezüglich der gemessenen inneren und äußeren Strahlendosen (Ri, Re) zu speichern.

14. Strahlenschutzschild gemäß einem der Ansprüche 1 bis 13, **dadurch gekennzeichnet, daß** die elektronischen Anzeigemittel (8) und die Steuerungsmittel (9) aus einer einstückigen elektronischen Vorrichtung bestehen.

15. Dosimetrisches System für ein Strahlenschutzschild (1), der dazu ausgelegt ist, den Strahlenschutz eines Bedieners vor ionisierenden Strahlen sicherzustellen,
wobei der Schild eine aus einem Strahlenschutzmaterial gefertigte Strahlenschutzwand (2) aufweist,
wobei die Strahlenschutzwand (2) zwei entgegengesetzte Seiten aufweist: eine Innenseite (25), gegenüber der der Bediener Position beziehen soll, und eine Außenseite (26), gegenüber der die Quelle ionisierender Strahlen positioniert sein soll,
wobei das dosimetrische System (5)
- erste dosimetrische Meßmittel (6), die gegenüber der Außenseite (26) angeordnet sind und dazu ausgelegt sind, eine an der Außenseite (26) empfangene äußere Strahlungsdosis (Re) zu messen,
- zweite dosimetrische Meßmittel (7), die gegenüber der Innenseite (25) angeordnet sind und dazu ausgelegt sind, eine an der Innenseite (25) empfangene äußere Strahlungsdosis (Ri) zu messen,
- elektronische Anzeigemittel (8), die dazu ausgelegt sind, für den Bediener bestimmte Informationen anzuzeigen, und
- mit den dosimetrischen Meßmitteln (6, 7) und den elektronischen Anzeigemitteln (8) verbundene Steuerungsmittel (9)
aufweist,
wobei die Steuerungsmittel (9) ein Steuerungsmodul (91) aufweisen, das dazu ausgelegt ist, auf den elektronischen Anzeigemitteln (8) mindestens auf die gemessenen inneren und äußeren Strahlendosen (Ri, Re) bezogene Informationen anzuzeigen.

## Claims

1. A radiation protection screen, that is adapted to ensure the radiation protection of an operator against ionising radiation,
said screen comprising a radiation protection wall (2) made of a radiation protection material,
said radiation protection wall having two opposite faces:
- an inner face (25), opposite which the operator is intended to position himself, and
- an outer face (26), opposite which a source of ionising radiation has to be placed,
**characterized in that** said radiation protection screen (1) is fitted with:
- first dosimetric measurement means (6), implanted opposite said outer face (26) and adapted to measure an external radiation dose (Re) received on the side of said outer face (26),
- second dosimetric measurement means (7), implanted opposite said inner face (25) and adapted to measure an internal radiation dose (Ri) received on the side of said outer face (25),
- electronic display means (8), adapted to display information for the operator, and
- control means (9), coupled to said dosimetric measurement means (6, 7) and said electronic display means (8),
said control means (9) including a control module (91) designed to display, on said electronic display means (8), information relating to at least said measured internal (Ri) / external radiation (Re) doses.

2. The radiation protection screen according to claim 1, **characterized in that** the control module (91) is designed to display, on said electronic display means (8), at least information chosen among:
- a value of the internal radiation dose (Ri),
- a value of the external radiation dose (Re),
- a differential radiation dose value, corresponding to the difference between said external radiation dose (Re) and said internal radiation dose (Ri), and
- an attenuation value, corresponding to the ratio between said external radiation dose (Re) and said internal radiation dose (Ri).

3. The radiation protection screen according to any one of claims 1 or 2, **characterized in that** the first dosimetric measurement means (6) are carried by the outer face (26) of the radiation protection wall (2) and the second measurement means (7) are carried by the inner face (25) of the radiation protection wall (2).

4. The radiation protection screen according to any one of claims 1 to 3, **characterized in that** the electronic display means (8) are carried by the inner face (25) of the radiation protection wall (2) and are arranged in such a way as to be visually accessible by the operator standing in front of said inner face (25).

5. The radiation protection screen according to any one of claims 1 to 4, **characterized in that** the control means (9) are coupled to said dosimetric measurement means (6, 7) and/or to said electronic display means (8), by means of wired or wireless connection means.

6. The radiation protection screen according to any one of claims 1 to 5, **characterized in that** it comprises means for the removable fastening of:
- said first dosimetric measurement means (6) opposite the outer face (26) of the radiation protection wall (2), and/or
- said second dosimetric measurement means (7) opposite the inner face (25) of the radiation protection wall (2), and/or
- the electronic display means (8) opposite the inner face (25) of the radiation protection wall (2).

7. The radiation protection screen according to claim 6, **characterized in that** said first dosimetric measurement means (6), and/or said second dosimetric measurement means (7), and/or said electronic display means (8), are each housed in a case removably fastened by magnetization to a metal wall of said screen (1).

8. The radiation protection screen according to claim 6, **characterized in that** said first dosimetric measurement means (6), and/or said second dosimetric measurement means (7), and/or said electronic display means (8), are housed in a case removably fastened by insertion into a support housing fastened to said screen (1).

9. The radiation protection screen according to any one of claims 1 to 8, **characterized in that** said first dosimetric measurement means (6), said second dosimetric measurement means (7) and said electronic display means (8) are powered by batteries carried by said screen (1).

10. The radiation protection screen according to claim 9, **characterized in that** said first dosimetric measurement means (6), said second dosimetric measurement means (7) and said electronic display means (8) are each housed in a case integrating a battery for the powering thereof, said cases comprising a suitable connection system for recharging said batteries from a power source.

11. The radiation protection screen according to any one of claims 1 to 10, **characterized in that** it comprises a radiation protection wall (2) comprising a front panel (21) extended by at least one lateral panel (22), said front (21) and lateral (22) panels extend over a height higher than the height of said operator in a standing position,
said first dosimetric measurement means (6) and said second dosimetric measurement means (7) being fastened to said lateral panel (22), on an area located opposite the head part of said operator,
and said electronic display means (8) being fastened on said front panel (21).

12. The radiation protection screen according to any one of claims 1 to 11, **characterized in that** the control means (9) includes an output for coupling to a remote computer.

13. The radiation protection screen according to any one of claims 1 to 12, **characterized in that** the control means (9) includes a memory module (92), designed to record the information relating to the measured internal (Ri) / external (Re) radiation doses.

14. The radiation protection screen according to any one of claims 1 to 13, **characterized in that** the electronic display means (8) and the control means (9) consist of a single-piece electronic device.

15. A dosimetric system, for a radiation protection screen (1) for providing radiation protection of an operator against ionising radiation,
said screen comprising a radiation protection wall (2) made of a radiation protection material, said radiation protection wall (2) comprising two opposite faces: an inner face (25), opposite which the operator has to stand, and an outer face (26), opposite which a source of ionising radiation has to be placed,
said dosimetric system (5) comprising:
- first dosimetric measurement means (6), intended to be implanted opposite said outer face (26) and adapted to measure an external radiation dose (Re) received on the side of said outer face (26),
- second dosimetric measurement means (7), intended be implanted opposite said inner face (25) and adapted to measure an internal radiation dose (Ri) received on the side of said inner face (25),
- electronic display means (8), adapted to display information for the operator, and
- control means (9), coupled to said dosimetric measurement means and said electronic display means (8),
said control means (9) including a control module (91) designed to display, on said electronic display means (8), information relating to at least said measured internal (Ri) / external radiation (Re) doses.
